# FASCICULE DE BREVET EUROPEEN

(11) **EP 2 311 501 B1**
(45) Date de publication et mention de la délivrance du brevet: **30.05.2018**
(21) Numéro de dépôt: 10183647.6
(22) Date de dépôt: 30.09.2010
(51) Int. Cl.: A61L 2/28

(54) **Dispositif de test pour contrôle d'appareil de stérilisation à prévide par la vapeur d'eau**
Prüfungsvorrichtung für die Kontrolle der Dampfqualität in einem Vakuumsterilisator
Testing device for controlling the vapour quality in a vacuum sterilizer

(30) Priorité: 02.10.2009 FR 0956895
(43) Date de publication de la demande: 20.04.2011
(73) Titulaire: Sterlab, 06220 Vallauris (FR)
(72) Inventeur: Martel, Paul, 83700, St-Raphaël (FR)
(74) Mandataire: Novagraaf Technologies

(56) Documents cités:
- EP-A1- 0 776 669
- WO-A1-93/21964
- WO-A1-95/32742
- WO-A1-97/12637

## Description

La présente invention a pour objet un dispositif de test pour contrôle d'appareil de stérilisation à prévide par la vapeur d'eau.

Le secteur technique de l'invention est le domaine de la fabrication de dispositifs de test aptes à contrôler la qualité du gaz stérilisant tel que la vapeur d'eau dans un appareil à prévide destiné à la stérilisation par cette vapeur d'eau et dont on veut donc déterminer l'efficacité du vide réalisé avant l'introduction de cette vapeur, puis la qualité de celle-ci une fois introduite dans l'enceinte.

La présente invention est particulièrement destinée aux tests d'appareils à prévide et de stérilisation par la vapeur d'eau tels que, mais non exclusivement, les autoclaves utilisés pour la stérilisation d'équipements médicaux et d'hôpitaux.

On connaît pour cela depuis 1961 la méthode de test dite de BOWIE-DICK qui a fait l'objet de diverses publications sans qu'il soit nécessaire donc de l'expliciter dans la présente demande : l'on pourra se référer utilement à diverses demandes de brevets antérieurs qui font référence à cette méthode, telle que la demande WO 93/21964 puliée le 11 novembre 1993, la demande US 4 579 715 publiée le 1er avril 1986 au nom de la Compagnie WARNER LAMBERT ou encore le brevet US 4 486 387 publié le 4 décembre 1984 au nom de la Société PROPPER MANUFACTURING Co ou même celui de la société MXM déposé le 20 octobre 2005 et publié sous le numéro FR 2 892 309, dont la présente introduction est en grande partie reprise.

On rappellera simplement que le but de ce test est de vérifier qu'il ne reste pas de gaz indésirable, en l'occurrence non condensables, dans l'enceinte de l'appareil stérilisateur : pour cela on utilise en général une encre sensible au gaz de stérilisation, telle qu'une composition chimique sensible à l'humidité, mais non aux gaz indésirables, et disposée suivant différents motifs sur une feuille de mesure glissée à l'intérieur soit d'un pack de matériau poreux soit dans un réceptacle creux fermé à une extrémité et ouvert à l'autre ; lequel réceptacle ou pack est placé dans l'appareil de stérilisation durant un cycle test qui est effectué, jusqu'à 134°C, à une fréquence prédéterminée, avant d'utiliser cet appareil pour stériliser des équipements. Un tel test, dont le résultat est vérifié donc a posteriori à la sortie de l'appareil, permet ainsi d'être sûr qu'il n'y avait plus de gaz indésirables tel que de l'air dans l'enceinte, car ces gaz pourraient se stratifier, créer des poches dont la température ne pourrait pas être contrôlée, ou se mélanger au gaz stérilisant, nuisant alors à la qualité de la stérilisation : si le test est positif, à savoir que l'encre sensible a changé de couleur sur toute la surface du motif, on peut en déduire qu'il n'y a eu pas de gaz indésirables pendant le cycle test et qu'il en sera de même lors des phases ultérieures de stérilisation d'équipements.

Diverses demandes de brevet ont été déposées pour protéger différentes particularités de dispositifs de test dont ceux déjà cités ci-dessus et également, parmi ceux à réceptacle dans la famille desquelles se situe la présente invention :
- le brevet US 6,630,352 publié le 1er juillet 1999 au nom de la société 3M INNOVATIVE PROPERTIES COMPANY qui décrit et revendique un dispositif complexe avec un réceptacle vertical dont l'ouverture est orientée vers le bas et entouré d'un radiateur externe pour évacuer la chaleur contenue dans la chambre du réceptacle qui est libre de toute charge et qui comprend deux capteurs de température situés contre et à l'extérieur de celle-ci pour en mesurer la température en deux points de sa paroi.
- Le brevet US 5,066,464 publié le 19 novembre 1991 au nom de la société PROPPER MANUFACTURING COMPANY, INC qui décrit et revendique un dispositif comprenant également un réceptacle mais disposé horizontalement, dont la section de l'ouverture est plus importante que celle de son extrémité fermée, la chambre dudit réceptacle comprenant un dissipateur de chaleur constitué d'une grande feuille de « champ » non tissé « bourrée » à la main dans cette chambre pour condenser la vapeur et un indicateur chimique sensible à l'humidité, tel que par changement de couleur, et disposé vers l'extrémité fermée de la chambre du réceptacle.

On retrouve dans ces deux exemples de réalisation le même principe de séparation d'air/vapeur par progression et condensation le long d'un réceptacle avec des dispositifs de dissipation de la chaleur pour condenser la vapeur d'eau et concentrer ainsi des gaz indésirables tels que l'air, qui sont soit sous forme de radiateurs placés à l'extérieur de la chambre soit sous forme de charges de remplissage placées à l'intérieur, et avec un dispositif de mesure soit électronique et placé respectivement à l'extérieur soit chimique et placé à l'intérieur.

Le problème posé et non complètement résolu dans aucun des documents connus de l'art antérieur, est d'une part d'obtenir des résultats fiables et les plus satisfaisants possible, et d'autre part de ne pas avoir à utiliser un pack de test que l'on doit ouvrir après usage comme dans la solution du brevet de la société MXM FR 2 892 309 évoqué précédemment, et enfin de simplifier les dispositifs de test à réceptacle tel que celui du brevet US 6,630,352 de la société 3M INNOVATIVE PROPERTIES COMPANY cité ci-dessus même si la solution de capteur électronique est plus facile d'usage que les dispositifs chimiques tels que celui du brevet US 5,066,464 de la société PROPPER MANUFACTURING COMPANY INC dont il faut retirer l'indicateur chimique pour en vérifie l'état a posteriori et dont de plus les résultats sont liés à l'interprétation que l'opérateur fait du virage colorimétrique.

Une solution au problème posé est un dispositif de test apte à contrôler la présence de gaz indésirables non condensables et donc la qualité de la vapeur d'eau stérilisant dans un appareil à prévide destiné à la stérilisation d'objets par cette vapeur, et comprenant une enveloppe portant des éléments du dispositif dont un réceptacle formant une chambre allongée fermée à son extrémité distale et ouverte à son extrémité proximale, et ladite chambre contenant une charge de remplissage occupant au moins une partie de son volume, et au moins un indicateur de présence du gaz indésirables étant disposé vers l'extrémité distale fermée de la chambre. et tel que :
- la surface de la paroi du réceptacle formant la chambre allongée est une surface de révolution dont la génératrice disposée au plus bas du dispositif en fonctionnement forme un angle α compris entre 0 et 90°, et même préférentiellement entre 0 et 30°, ou même entre 0 et 10°, par rapport et en-dessous de l'horizontale, depuis le fond fermé du réceptacle vers son extrémité proximale ouverte,
- l'indicateur de présence de gaz indésirables est un capteur de température électronique de tout type, lequel est apte à permettre de calculer la proportion de ce gaz non condensable indésirable dans l'atmosphère de l'extrémité distale de la chambre,
- la charge de remplissage est telle qu'elle comporte au moins, dans la position de fonctionnement de la chambre du réceptacle qui la contient, un chemin permettant un écoulement par gravité de l'eau condensée dans la chambre du réceptacle à partir de la vapeur d'eau constituant principalement l'atmosphère de celle-ci, vers l'ouverture de l'extrémité proximale,
et selon l'invention :
- l'enveloppe a une grande inertie thermique au moins autour de l'extrémité distale de la chambre où est disposé l'indicateur de présence de gaz indésirables, l'épaisseur au moins de cette enveloppe étant telle pour cela que son diamètre externe est compris entre le double et le quadruple du diamètre interne du réceptacle isolé ainsi de l'extérieur.

Dans un mode préférentiel de réalisation la charge de remplissage est constitué d'un ou d'au moins un élément rigide et de préférence de plusieurs éléments discrets, ayant préférentiellement une forme extérieure géométrique régulière telle qu'une sphère, lesdits éléments discrets pouvant être ainsi des billes en matériau rigide, tel qu'en acier, en verre....

Dans un autre mode préférentiel de réalisation, le réceptacle est un cylindre dont l'axe AA' forme, en fonctionnement, un angle α compris entre 0 et 90° par rapport à l'horizontale, le centre de l'ouverture étant au moins au même niveau ou en dessous de celui de l'extrémité distale fermée.

Le résultat est un nouveau type de dispositif de test répondant au problème posé et aux divers inconvénients des dispositifs actuels puisque d'une part on dispose ainsi d'un dispositif de test assez simple, avec une mesure électronique évitant d'avoir à changer à chaque manipulation l'ensemble de l'intérieur du réceptacle (ou même à remplacer celui-ci) et ayant de plus des résultats de mesure très améliorés qui ont été constatés d'une manière du reste assez surprenante par des essais.

On rappellera qu'avant d'arriver à l'équilibre thermique, la température d'un mélange vapeur et de gaz indésirables tel que l'air, est inférieure à la température de la vapeur seule : en mesurant ainsi la température dans l'atmosphère de l'extrémité distale à l'intérieur de la chambre du réceptacle suivant l'invention, si celle-ci s'avère différente de la température de la vapeur remplissant l'atmosphère extérieure au dispositif de l'invention, il y a nécessairement concentration des gaz indésirables et ceux-ci sont bien présents dans l'appareil de stérilisation à prèvide que l'on veut tester ; et plus la différence de température est importante, plus cela signifie qu'il y a une grande proportion de gaz non condensable dans la vapeur (tel que cela peut arriver si le prévide a été défaillant, ou s'il y a eu un apport de vapeur chargé de tels gaz, ou s'il y a une fuite...).

Ainsi, lors d'essais, dont les résultats sont donnés ci-après, on a mesuré la différence de températures ci-dessus dans différentes configurations de dispositif de test et chacun suivant deux situations, respectant les conditions de tests définis par la norme ISO 11140-4, l'une en l'absence d'air (condition de défaillance zéro) et l'autre après des injections d'air (caractérisant les défaillances: : le critère d'un bon fonctionnement du dispositif de test étant alors de relever une différence de température quasi nulle dans la première situation et importante dans la deuxième, et de toute façon en ce cas avec un écart significatif (soit de plus de 15°C) entre les deux différences pour éliminer les risques de « bruits de fond ».

Il a été ainsi effectué quatre séries d'essais :
Essais 1 : état de l'art antérieur avec différentes dimensions de chambre, cylindrique de révolution, sans charge intérieure, comme dans le brevet US 6 630 352 cité précédemment, et placé horizontalement ; cependant avec des diamètres trop faibles, tel que le montre le premier essai de ces essais 1 avec un diamètre de 10 mm pour une longueur de chambre de 100 mm, l'écart de température, même en absence d'air, est beaucoup trop important (67°C dans cet exemple) et la comparaison avec un écart en présence d'air ne peut pas avoir de signification; on écarte ainsi pour les autres essais les petits diamètres de 10 et 12 mm en ne gardant que celui de 14 mm avec une longueur de 70 mm (les plus grandes longueurs donnant en effet des écarts de température, même en présence d'air, non négligeables sont aussi à écarter : voir les essais 2 avec d'un côté 80 mm de longueur pour 14 mm de diamètre et, d'un autre côté, 70 mm de longueur pour 14 mm de diamètre) ;
Essais 2 : essais avec des charges rigides (résultat très proche avec des billes en verre de 3 mm de diamètre, granules en plastiques, billes en acier) dans deux chambres de longueur différente, cylindrique de révolution, placées horizontalement ;
Essais 3 : essais avec des charges rigides et la chambre optimale des essais 2, suivant différentes inclinaisons par rapport à l'horizontale;

Une quatrième série d'essais avec des charges souples a donné des résultats meilleurs que dans la première série d'essais mais avec une grande dispersion de mesures que nous n'avons pas reproduits dans le tableau ci-après (référencé figure 9).

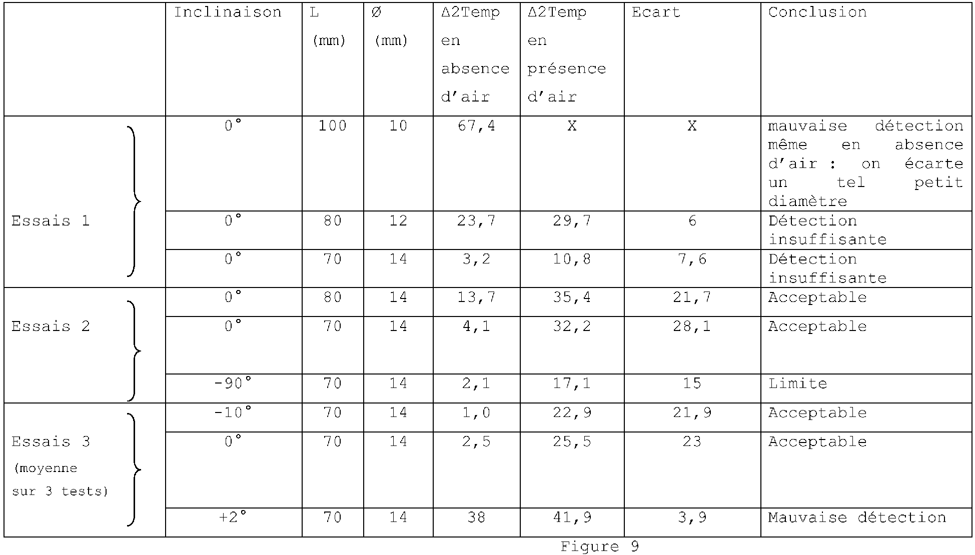

Ces résultats ont été très surprenants car au-delà de ce qui pouvait être espéré dans le choix des configurations : ils ont ainsi montré d'une manière inattendue l'importance, pour avoir des écarts de température qualifiés d'acceptables :
- d'une part, de l'inclinaison de la chambre du réceptacle alors que dans l'art antérieur on relève des dispositifs aussi bien verticaux qu'horizontaux ou sans recommandation sur la nécessité de contrôler cette inclinaison alors que lorsque l'on pose quand il est autonome un dispositif sur un support, celui-ci peut provoquer une inclinaison suivant sa propre configuration et,
- d'autre part, de la présence d'une charge (ayant des caractéristiques préférentielles telles qu'indiquées ci-après) dans la chambre alors que l'art antérieur enseigne des dispositifs sans charge ou avec une charge intérieure souple mais sans qu'il soit possible à l'homme du métier de savoir si celle-ci joue un rôle essentiel et d'autant plus en liaison avec l'inclinaison ; en effet, les essais ont bien montré qu'une inclinaison vers le bas de l'ouverture du réceptacle avec une charge n'influence que très faiblement la qualité de la détection d'air qui reste excellente, alors qu'à l'opposé, une inclinaison vers le haut, même infime, et pouvant être provoquée par un mauvais positionnement du dispositif sur son support (ce qui doit être ainsi d'autant plus précisé, dans la présente invention, aux utilisateurs, si la chambre du dispositif est horizontale par construction ; et pour limiter un tel risque de mauvais positionnement, il peut être préférable, tel que décrit ci-après, de disposer d'une chambre dont la paroi inférieure est inclinée par construction vers le bas, même si le dispositif est destiné à être normalement positionné horizontalement) génère des situations où la capacité de distinguer la présence de l'absence d'air disparaît.

Une caractéristique essentielle suivant l'invention s'avère donc bien que cette charge et la position, ou la forme, du réceptacle qui la contient doivent permettre un écoulement par gravité de l'eau condensée dans la chambre vers l'ouverture de celle-ci, et suivant un mode de réalisation préférentiel, la charge est constitué d'un élément rigide ou mieux de plusieurs éléments discrets rigides, telles que des billes de verre, de plastique ou d'acier.

Les figures ci-jointes représentent des exemples de réalisation de divers dispositifs suivant l'invention mais ceux-ci ne sont pas limitatifs.
La figure 1 représente un dispositif suivant l'invention, autonome et dont l'enveloppe 8 est placée horizontalement sur un support 9 horizontal, tel que la paroi inférieure de l'enceinte de l'appareil de stérilisation à prévide par la vapeur d'eau et que l'on veut tester : ce dispositif comprend un alésage ou réceptacle de forme cylindrique d'axe horizontal, avec une charge rigide, et un capteur disposé à l'intérieur au fond de ce réceptacle.
La figure 2 est un dispositif comprenant les mêmes éléments que sur la figure 1, mais dont l'axe de la chambre est franchement incliné, l'ouverture vers le bas, même si le dispositif est lui-même disposé horizontalement.
La figure 3 est un dispositif suivant l'invention disposé comme sur les figures 1 et 2, mais dans lequel l'alésage est de forme conique.
La figure 4 est un dispositif suivant l'invention du type de celui de la figure 1 mais avec une charge rigide poreuse.
La figure 5 est un dispositif suivant l'invention du type de celui de la figure 3 avec une charge rigide constituée d'éléments discrets.
La figure 6 est un dispositif suivant l'invention du type de celui de la figure 1 dans lequel la charge rigide est constituée d'éléments discrets.
La figure 7 est un dispositif suivant l'invention du type de celui de la figure 1 avec un capteur de température (indicateur de présence de gaz indésirables, tel que l'air) disposé à l'intérieur et sur la paroi latérale du réceptacle.
La figure 8 représente un dispositif suivant l'invention du type de celui de la figure 1 mais dont l'enveloppe 8 est disposée suivant une inclinaison maximale de 90° par rapport à l'horizontale du support 9, l'ouverture de la chambre vers le bas.

Dans tous les modes de réalisation, tels que représentés ici et d'autres possibles ayant les caractéristiques de l'invention, le dispositif suivant l'invention comprend un réceptacle 1 formant une chambre allongée 5 fermée à son extrémité distale 1₁ et ouverte à son extrémité proximale 1₂, et ladite chambre 5 contient une charge de remplissage 3 occupant au moins une partie de son volume. Dans les exemples de réalisation représentés sur les figures jointes, les extrémités 1₁ et 1₂ sont disposées directement à l'opposé l'une de l'autre suivant une même direction, mais dans d'autres modes de réalisation le réceptacle 1 pourrait être courbe et ses extrémités, même si on pourrait toujours les considérer à l'opposé l'une de l'autre par rapport au volume de la chambre 5 alors non droite et qui les sépare, ne seraient plus directement en vis-à-vis (elles pourraient être par exemple dans un même plan, comme dans le cas où la chambre serait en forme d'un U).

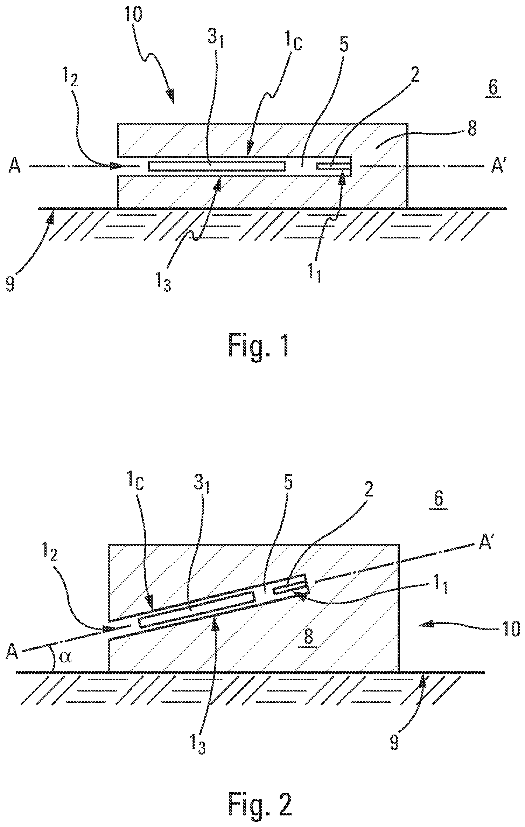

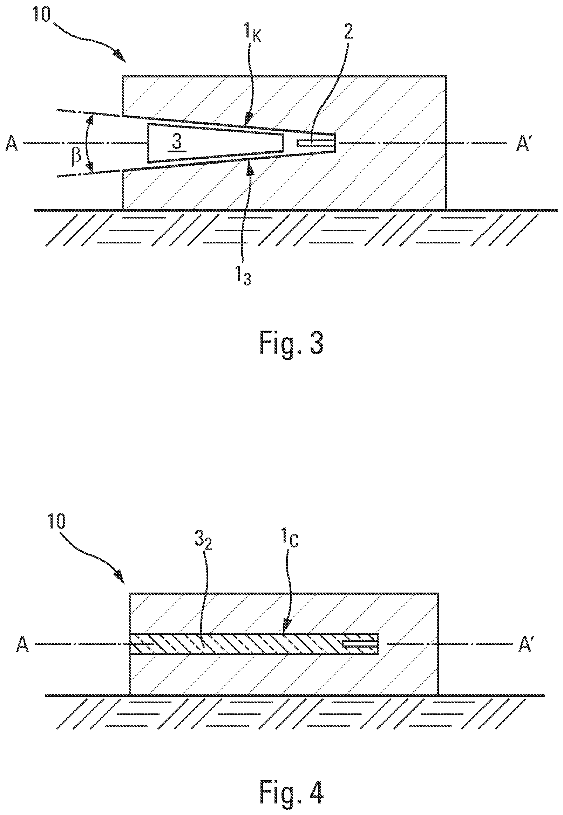

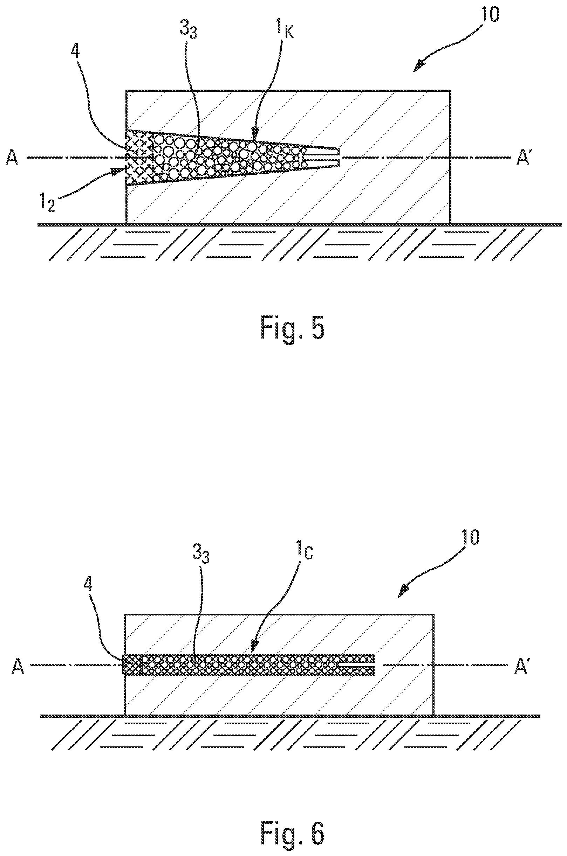

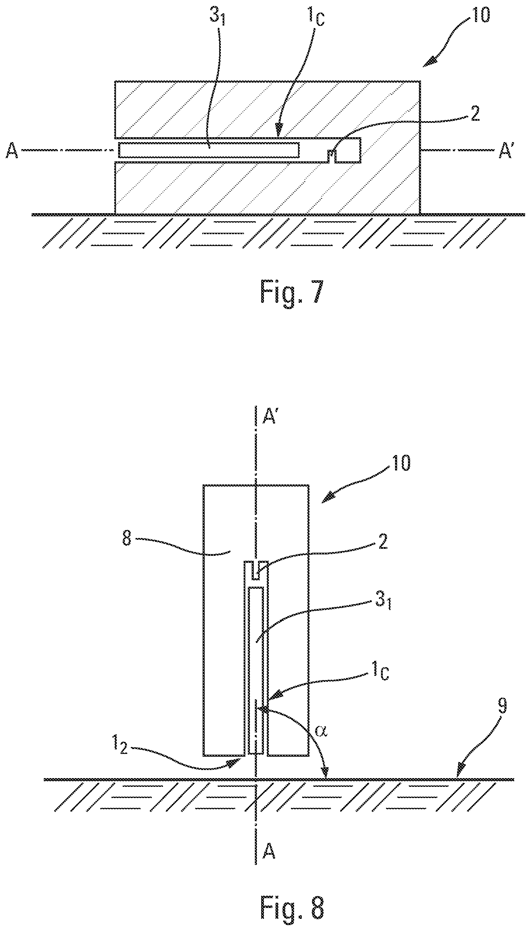

La charge de remplissage 3 est telle qu'elle comporte au moins, dans la position de fonctionnement de la chambre 5, un chemin permettant un écoulement par gravité de l'eau condensée dans la chambre 5 à partir de la vapeur d'eau constituant principalement l'atmosphère de celle-ci, vers l'ouverture de l'extrémité proximale 1₂. Cette caractéristique est obtenue dans tous les exemples ci-joints par la combinaison de deux critères :
- d'une part, la position de fonctionnement de la chambre 5 doit être telle que la partie inférieure 1₃ de la paroi latérale de l'alésage ou réceptacle 1, disposée en fonctionnement au plus bas du dispositif, est toujours au moins horizontale (suivant l'exemple des figures 1, 4, 6 et 7) ou inclinée depuis le fond 1₁ du réceptacle vers son ouverture 1₂, (tel que sur la figure 2 par l'inclinaison de l'axe A,A' de l'alésage ou réceptacle 1, si celui-ci est un cylindre 1c, en particulier de révolution, ou par le choix d'un alésage en forme de cône 1k d'angle au sommet β et dans lequel en fonctionnement aucune de ses génératrices ne fait un angle vers le haut avec l'horizontale, supérieur à β, tel que représenté sur les figures 3 et 5, ou encore en inclinant l'ensemble du dispositif suivant un angle α pouvant aller jusqu'à 90° par rapport à l'horizontale, l'ouverture 1₂ de l'alésage étant dirigée vers le bas, comme sur la figure 8),
- d'autre part, le choix de la charge de remplissage qui peut être de préférence une charge rigide tel qu'un élément en matériau monobloc 3₁ non poreux à la vapeur d'eau et aux gaz indésirables tel que l'air, et qui obture partiellement toute section de l'alésage ou réceptacle 1, comme sur la représentation des figures 1 à 3, et 7 et 8, mais aussi un élément monobloc 3₂ constitué d'un matériau de préférence rigide, poreux à la vapeur d'eau et aux gaz indésirables tel que l'air et pouvant alors occuper complètement la section de l'alésage, tel que représenté sur la figure 4, ou encore dans un mode préférentiel de réalisation une pluralité d'éléments discrets 3₃ et pouvant remplir complètement la section de l'alésage 1, tels que ceux cités ci-dessus lors des essais (tels que des billes en acier ou en verre de préférence de 2 à 3 mm de diamètre, ou des granulés de polypropylène tels que ceux utilisés pour réaliser des produits par injection) comme représenté sur les exemples des figures 5 et 6 ; en ce cas, un tampon de type en mousse ou autre matériau poreux, laissant passer tous gaz indésirables et la vapeur mais bien sûr pas les éléments discrets 3₃, ferme l'extrémité proximale 1₂ de la chambre 5.

Lors des essais, tel que décrit précédemment, il a été constaté que les billes de métal ou en acier sont des éléments discrets pouvant constituer la charge de remplissage et qui offrent le plus de régularité sur l'appréciation de la pénétration de la vapeur, mais ces billes provoquent une augmentation de l'inertie thermique, ce qui peut être contraignant. Par ailleurs, les billes ou granulés en plastique, à l'inverse, ont une très faible inertie thermique et réagissent plutôt bien à la détection de l'air ; de plus elles sont peu onéreuses, mais des tests plus approfondis ont néanmoins montré qu'elles présentaient un caractère instable sur du long terme. En conséquence, le choix préférentiel peut se porter sur des billes en verre dont les essais ont confirmé qu'elles offraient le meilleur compromis.

Ainsi, en combinaison de la position et de la forme du réceptacle tel que décrit précédemment avec une charge de remplissage suivant les caractéristiques ci-dessus, il y a toujours un chemin qui permet un écoulement par gravité de l'eau condensée dans la chambre 5 vers l'ouverture de l'extrémité proximale 1₂.

Le dispositif de test suivant l'invention comprend également un indicateur 2 de présence de gaz indésirables, disposé vers l'extrémité distale 1₁ fermée de la chambre 5, et qui, suivant l'invention, est un capteur électronique de température apte à permettre de calculer la proportion de ce gaz non condensable indésirable tel que l'air dans l'atmosphère de l'extrémité proximale 1₂ de la chambre 5.

Ce capteur électronique 2 de température est positionné soit de préférence à l'intérieur du volume de la chambre 5 pour être en contact direct avec l'atmosphère de celle-ci, soit dans l'épaisseur de l'enveloppe 8 mais proche de la paroi du réceptacle 1 pour donner des valeurs de température, certes pouvant être alors sensiblement différentes de celles de la température intérieure de l'extrémité distale 1₁ de la chambre 5, mais permettant toutefois une discrimination et une fidélité suffisantes de mesure d'écart de température comme défini précédemment.

Cette mesure de température permet, comme expliqué précédemment, de déterminer si le dispositif suivant l'invention a concentré ou non des gaz indésirables et donc de savoir si l'enceinte dans laquelle il a été placé a bien été vidé de tous gaz indésirables tel que de l'air avant son remplissage par de la vapeur d'eau : la température relevée par ce capteur 1 est enregistrée dans un dispositif électronique comprenant une mémoire 7 logée dans l'enveloppe 8 du dispositif.

L'enveloppe 8 doit avoir une grande inertie thermique qui peut être obtenue par toute configuration, comme l'homme du métier peut la concevoir (tel que par exemple en réalisant un boîtier en matériau en plastique résistant à la chaleur, soit jusqu'à 135°C, avec une longueur de l'enveloppe égale au double de celle du réceptacle et une grande épaisseur de cette enveloppe tel qu'un diamètre externe compris entre le double et le quadruple du diamètre interne du réceptacle qu'il isole ainsi de l'extérieur) : cette grande inertie thermique doit concerner au moins la partie de l'enveloppe entourant l'extrémité 1₁ distale de la chambre 5, extrémité où est situé le capteur de température 2, pour que celui-ci ne subisse pas l'influence de la température extérieure, la chaleur n'étant alors nécessairement amenée suivant la présente invention que par l'extrémité proximale du réceptacle.

Le dispositif suivant l'invention peut être soit autonome, l'enveloppe 8 étant alors un boîtier indépendant et mobile comme représenté sur les figures, soit intégré à l'appareil à prévide, l'enveloppe 8 pouvant être alors un élément de l'enceinte de l'appareil.

Ladite mémoire enregistre cette température pendant la totalité du cycle de mesure et le calcul du pourcentage de gaz non condensable prend en compte une ou plusieurs valeurs de cette température pendant un ou plusieurs moments de ce cycle; la mesure de la température relevée par le capteur interne 2 est comparée soit à celle de la température externe mesurée et enregistrée en même temps par un capteur disposé à l'extérieur de la chambre 5, soit à une température prédéterminée de référence: la comparaison par différence entre les deux températures permet d'évaluer le pourcentage de gaz non condensable tel que l'air pouvant se trouver dans l'enceinte considérée, comme explicité lors de la présentation des essais décrits précédemment.

Ce capteur de température peut être disposé contre le fond 1₁ de la chambre 5 à l'intérieur de l'alésage, ou réceptacle 1, tel que représenté sur l'ensemble des figures 1 à 6 et 7 mais également peut être disposé contre la paroi latérale 1₃ de la chambre 5, à l'intérieur du réceptacle 1 mais toujours vers l'extrémité distale 1₁ de celui-ci. Il pourrait être disposé également dans les mêmes zones de la chambre 5 mais, comme indiqué précédemment, dans l'épaisseur de l'enveloppe 8 et proche de la paroi de la chambre 5.

On peut noter que le dispositif suivant l'invention peut être utilisé pour servir de « piège » à air pendant des cycles de stérilisation opérationnels, et donc sans avoir nécessairement besoin de comporter de capteur de température puisqu'il n'est pas nécessaire ni utile alors pour cette application de faire une mesure de test de qualité du gaz stérilisant, l'objectif étant d'améliorer seulement la stérilisation en cas de mauvais fonctionnement qui apporterait de l'air dans l'enceinte (et qui aurait été éventuellement mesurée précédemment par un test, ou simplement par précaution) : dans une telle utilisation le dispositif comporte une grande chambre (de dimensions plus importantes que celles décrites précédemment, puisque dans cet objectif de « piège » à air il faut pouvoir disposer d'un volume important pour y concentrer et garder le plus d'air possible) ; une telle grande chambre peut avoir un diamètre de 20 à 50 mm pour une longueur de 100 à 150 mm par exemple, avec une inertie thermique et donc une isolation importante (cependant moindre que dans le cas d'un dispositif de test puisqu'ici l'influence de la température extérieure est moins critique et nécessite donc moins d'inertie thermique) : une épaisseur de 10 à 20 mm de paroi isolante de l'enveloppe peut être suffisante. Un tel dispositif comporte nécessairement une charge de remplissage, telle que celle précédemment décrite suivant l'invention, et occupant au moins une partie du volume de la chambre du réceptacle, celui-ci devant être disposé dans les mêmes conditions que celles décrites pour le dispositif de test.

## Revendications

1. Dispositif de test apte à contrôler la présence de gaz indésirables non condensables et donc la qualité de la vapeur d'eau stérilisant dans un appareil à prévide destiné à la stérilisation par cette vapeur, et comprenant une enveloppe (8) portant des éléments du dispositif dont un réceptacle (1) formant une chambre allongée (5) fermée à son extrémité distale (1₁) et ouverte à son extrémité proximale (1₂) et dont la surface de paroi est une surface de révolution dont la génératrice disposée au plus bas du dispositif en fonctionnement forme un angle α compris entre 0 et 90° par rapport et en-dessous de l'horizontale, depuis le fond fermé de réceptacle vers son extrémité proximale ouverte (1₂), et ladite chambre (5) contenant :
- une charge de remplissage (3) occupant au moins une partie de son volume et telle qu'elle comporte au moins, dans la position de fonctionnement de la chambre 5, un chemin permettant un écoulement par gravité de l'eau condensée dans la chambre (5) à partir de la vapeur d'eau constituant principalement l'atmosphère de celle-ci, vers l'ouverture de l'extrémité proximale (1₂), et
- au moins un indicateur (2) de présence de gaz indésirables étant disposé vers l'extrémité distale (1₁) fermée de la chambre (5) et qui est un capteur électronique de température lequel est apte à permettre de calculer la proportion de ce gaz non condensable indésirable dans l'atmosphère de l'extrémité distale (1₁) de la chambre (5),
- l'enveloppe (8) ayant une grande inertie thermique au moins autour de l'extrémité (1₁) distale de la chambre (5) où est situé le capteur de température (2), l'épaisseur au moins de cette enveloppe (8) étant telle pour cela que son diamètre externe est compris entre le double et le quadruple du diamètre interne du réceptacle (1) isolé ainsi de l'extérieur.

2. Dispositif de test selon la revendication 1, **caractérisé en ce que** la charge de remplissage (3) est constituée d'au moins un élément rigide.

3. Dispositif de test selon l'une quelconque des revendications 1 ou 2, **caractérisé en ce que** la surface de la paroi du réceptacle (1) formant la chambre allongée (5) est une surface de révolution.

4. Dispositif de test selon la revendication 1 ou 2, **caractérisé en ce que** le réceptacle (1) est un cylindre dont l'axe AA' forme, en fonctionnement, un angle α compris entre 0 et 90° par rapport à l'horizontale, le centre de l'ouverture (1₂) étant au moins au même niveau ou en dessous de celui de l'extrémité distale fermée 1₁).

5. Dispositif de test suivant la revendication 4, **caractérisé en ce que** le réceptacle (1) est un cylindre de révolution.

6. Dispositif de test suivant la revendication 3, **caractérisé en ce que** la surface de révolution est celle d'un cône d'angle au sommet β dans lequel, en fonctionnement, aucune de ses génératrices ne fait un angle vers le haut avec l'horizontale supérieur à β.

7. Dispositif de test suivant l'une quelconque des revendications 1 à 6, **caractérisé en ce que** le capteur de température est disposé contre le fond (1₁) de la chambre (5), à l'intérieur du réceptacle (1).

8. Dispositif de test suivant l'une quelconque des revendications 1 à 6, **caractérisé en ce que** le capteur de température est disposé contre la paroi latérale (1₃) de la chambre (5), à l'intérieur du réceptacle (1), vers l'extrémité distale (1₁) de celui-ci.

9. Dispositif de test suivant l'une quelconque des revendications 1 à 8, **caractérisé en ce que** la charge de remplissage (3) est constituée d'un élément monobloc en matériau poreux (3₂) à la vapeur d'eau et aux gaz indésirables.

10. Dispositif de test suivant la revendication 2, **caractérisé en ce que** la charge de remplissage (3) est constituée d'un élément monobloc rigide (3₁) en matériau non poreux à la vapeur d'eau et aux gaz indésirables et obturant partiellement toute section du réceptacle (1).

11. Dispositif de test suivant l'une quelconque des revendications 1 à 8, **caractérisé en ce que** la charge de remplissage (3) est constituée d'une pluralité d'éléments discrets (3₃).

12. Dispositif de test suivant la revendication 11, **caractérisé en ce que** les éléments discrets (3₃) sont des billes en matériau rigide.

13. Dispositif de test suivant l'une quelconque des revendications 1 à 12, **caractérisé en ce qu'**il est apte à être intégré à l'appareil à prévide.

## Patentansprüche

1. Prüfvorrichtung, welche in der Lage ist, das Vorhandensein von unerwünschten nicht kondensierbaren Gasen und folglich die Qualität des sterilisierenden Wasserdampfes in einem Vakuumgerät, das zum Sterilisieren durch diesen Dampf bestimmt ist, zu kontrollieren, und eine Ummantelung (8) umfassend, welche Elemente der Vorrichtung trägt, darunter eine Aufnahme (1), welche eine längliche Kammer (5) bildet, die an ihrem distalen Ende (1₁) geschlossen und an ihrem proximalen Ende (1₂) offen ist, und deren Wandfläche eine rotationssymmetrische Fläche ist, deren Erzeugende, im Betrieb ganz unten an der Vorrichtung angeordnet, einen Winkel α im Bereich zwischen 0 und 90° in Bezug zu und unterhalb der Horizontalen, vom geschlossenen Boden der Aufnahme zu ihrem proximalen offenen Ende (1₂) hin bildet, und wobei die Kammer (5) enthält:
- eine Füllung (3), die mindestens einen Teil ihres Volumens einnimmt und derart, dass sie mindestens in der Betriebsstellung der Kammer (5) einen Pfad umfasst, der ein Abfließen durch Schwerkraft des in der Kammer (5) kondensierten Wassers aus dem Wasserdampf, der im Wesentlichen die Atmosphäre derselben ausmacht, zur Öffnung des proximalen Endes (1₂) hin ermöglicht, und
- mindestens einen Indikator (2) des Vorhandenseins von unerwünschten Gasen, der zum geschlossenen distalen Ende (1₁) der Kammer (5) hin angeordnet ist und der ein elektronischer Temperatursensor ist, welcher in der Lage ist, das Berechnen des Anteils dieses unerwünschten nicht kondensierbaren Gases in der Atmosphäre des distalen Endes (1₁) der Kammer (5) zu ermöglichen,
- wobei die Ummantelung (8) eine große Wärmeträgheit mindestens um das distale Ende (1₁) der Kammer (5) herum aufweist, an dem der Temperatursensor (2) liegt, wobei die Dicke mindestens dieser Ummantelung (8) derart ist, dass ihr Außendurchmesser im Bereich zwischen dem Zweifachen und dem Vierfachen des Innendurchmessers der Aufnahme (1), welche so von der Außenseite isoliert ist, beträgt.

2. Prüfvorrichtung nach Anspruch 1, **dadurch gekennzeichnet, dass** die Füllung (3) aus mindestens einem starren Element besteht.

3. Prüfvorrichtung nach einem der Ansprüche 1 oder 2, **dadurch gekennzeichnet, dass** die Fläche der Wand der Aufnahme (1), welche die längliche Kammer (5) bildet, eine rotationssymmetrische Fläche ist.

4. Prüfvorrichtung nach Anspruch 1 oder 2, **dadurch gekennzeichnet, dass** die Aufnahme (1) ein Zylinder ist, dessen Achse AA' im Betrieb einen Winkel α im Bereich zwischen 0 und 90° in Bezug zur Horizontalen bildet, wobei der Mittelpunkt der Öffnung (1₂) mindestens auf derselben Ebene oder unterhalb derjenigen des geschlossenen distalen Endes (1₁) liegt.

5. Prüfvorrichtung nach Anspruch 4, **dadurch gekennzeichnet, dass** die Aufnahme (1) ein rotationssymmetrischer Zylinder ist.

6. Prüfvorrichtung nach Anspruch 3, **dadurch gekennzeichnet, dass** die rotationssymmetrische Fläche diejenige eines Kegels mit einem Scheitelwinkel β ist, wobei im Betrieb keine ihrer Erzeugenden einen aufwärts gerichteten Winkel zur Horizontalen von größer als β formt.

7. Prüfvorrichtung nach einem der Ansprüche 1 bis 6, **dadurch gekennzeichnet, dass** der Temperatursensor gegen den Boden (1₁) der Kammer (5) auf der Innenseite der Aufnahme (1) angeordnet ist.

8. Prüfvorrichtung nach einem der Ansprüche 1 bis 6, **dadurch gekennzeichnet, dass** der Temperatursensor gegen die Seitenwand (1₃) der Kammer (5) auf der Innenseite der Aufnahme (1) zum distalen Ende (1₁) derselben hin angeordnet ist.

9. Prüfvorrichtung nach einem der Ansprüche 1 bis 8, **dadurch gekennzeichnet, dass** die Füllung (3) aus einem einteiligen Element aus für den Wasserdampf und für die unerwünschten Gase porösem Material (3₂) besteht.

10. Prüfvorrichtung nach Anspruch 2, **dadurch gekennzeichnet, dass** die Füllung (3) aus einem einteiligen starren Element (3₁) aus nicht für den Wasserdampf und für die unerwünschten Gase porösem Material besteht und teilweise jeden Abschnitt der Aufnahme (1) verschließt.

11. Prüfvorrichtung nach einem der Ansprüche 1 bis 8, **dadurch gekennzeichnet, dass** die Füllung (3) aus einer Vielzahl von diskreten Elementen (3₃) besteht.

12. Prüfvorrichtung nach Anspruch 11, **dadurch gekennzeichnet, dass** die diskreten Elemente (3₃) Kugeln aus starrem Material sind.

13. Prüfvorrichtung nach einem der Ansprüche 1 bis 12, **dadurch gekennzeichnet, dass** sie in der Lage ist, in das Vakuumgerät integriert zu werden.

## Claims

1. Test device capable of checking for the presence of non-condensable undesirable gases, and therefore the quality of the sterilizing steam in a pre-vacuum apparatus intended for sterilization with said steam, and including an envelope (8) carrying elements of the device, including a receptacle (1) forming an elongate chamber (5) closed at its distal end (1₁) and open at its proximal end (1₂) and of which the surface of the wall is a surface of revolution, of which the generatrix arranged at the very bottom of the operating device forms an angle α between 0 and 90° with respect to and below the horizontal, from the closed end of the receptacle to the proximal open end (1₂), and in which said chamber (5) contains:
- a filling load (3) occupying at least a portion of its volume, and such that it comprises at least, in the operating position of the chamber 5, one path enabling a flow by gravity of the water condensed in the chamber (5) from the steam mainly forming the atmosphere of same, to the opening of the proximal end (1₂), and
- at least one undesirable gas presence indicator (2) arranged toward the distal closed end (1₁) of the chamber (5) and which is an electronic temperature sensor, which is able to allow calculation of the proportion of said non-condensable undesirable gas in the atmosphere at the distal end (1₁) of the chamber (5),
- the envelope (8) having a high thermal inertia at least around the distal end (1₁) of the chamber (5), where the temperature sensor (2) is located, the thickness at least of this envelope (8) being such as its external diameter is between twice and four times the internal diameter of the receptacle (1), which it thus insulates from the outside.

2. Test device according to claim 1, **characterized in that** the filling load (3) consists of at least one rigid element.

3. Test device according to either one of claims 1 or 2, **characterized in that** the surface of the wall of the receptacle (1) forming the elongate chamber (5) is a surface of revolution.

4. Test device according to claim 1 or 2, **characterized in that** the receptacle (1) is a cylinder of which the axis AA' forms, in operation, an angle α between 0 and 90° with respect to the horizontal, in which the center of the opening (1₂) is at least at the same level or below that of the distal closed end (1₁).

5. Test device according to claim 4, **characterized in that** the receptacle 1() is a cylinder of revolution.

6. Test device according to claim 3, **characterized in that** the surface of revolution is that of a cone with a top angle β in which, in operation, none of its generatrices forms an angle upwards with the horizontal greater than β.

7. Test device according to any one of claims 1 to 6, **characterized in that** the temperature sensor is arranged against the closed end (1₁) of the chamber (5), inside the receptacle (1).

8. Test device according to any one of claims 1 to 6, **characterized in that** the temperature sensor is arranged against the side wall (1₃) of the chamber (5), inside the receptacle, toward the distal end (1₁) of same.

9. Test device according to any one of claims 1 to 8, **characterized in that** the filling load (3) consists of a one-piece element made of a material porous (3₂) to steam and to undesirable gases.

10. Test device according to claim 2, **characterized in that** the filling load (3) consists of a rigid one-piece element (3₁) made of a material that is non-porous to steam and to undesirable gases and partially closing off every section of the receptacle (1).

11. Test device according to any one of claims 1 to 8, **characterized in that** the filling load (3) consists of a plurality of separate discrete elements (3₃).

12. Test device according to claim 11, **characterized in that** the separate discrete elements (3₃) are small balls made of a rigid material.

13. Test device according to any one of claims 1 to 12, **characterized in that** it is capable of being integrated in the pre-vacuum apparatus.
